# EUROPEAN PATENT APPLICATION

(11) **EP 0 841 403 A2**
(43) Date of publication of application: **13.05.1998**
(21) Application number: 97118573.1
(22) Date of filing: 25.10.1997
(51) Int. Cl.: C12Q 1/04

(54) **Coloring composition for microorganisms, filter tool for entrapping bacteria, and kit for measuring the number of bacteria**

(30) Priority: 08.11.1996 JP 296585/96; 03.02.1997 JP 20134/97
(71) Applicant: IDEMITSU KOSAN COMPANY LIMITED, Tokyo 100 (JP)
(72) Inventor: Sato, Mikio, Sodegaura-shi, Chiba, 299-02 (JP); Ito, Tomomi, Sodegaura-shi, Chiba, 299-02 (JP); Kadota, Akihiko, Sodegaura-shi, Chiba, 299-02 (JP)
(74) Representative: Türk, Gille, Hrabal, Leifert

(57) **Abstract**

Provided are a coloring composition and a coloring method for microorganisms, and a method for storing the composition. Using the composition, the number of microorganisms in samples can be measured rapidly and in a simplified manner, and the degree of coloration of the colored microorganisms varies little with the lapse of time. The composition has good storage stability. Also provided is a filter tool for entrapping bacteria, with which only bacteria can be entrapped rapidly and in a simplified manner from samples containing bacteria and animal cells or vegetable cells. The filter tool is utilized in a kit and a method for measuring the number of bacteria in samples. Further provided are a kit and a method for measuring the number of bacteria, which do not require any special equipment and any expert technical knowledge. Using the kit, the number of bacteria in samples can be measured rapidly and in a simplified manner through one-stage filtration. The coloring composition comprises a dye and a surfactant, and is controlled to have a pH of from 4 to 6 or is used in a controlled pH range of from 4 to 6. The composition comprising a dye and a surfactant is stably stored in a pH condition ranging from 4 to 6; or the composition comprising a dye is stored in a pH condition ranging from 4 to 6, and a surfactant is added thereto before use. The filter tool comprises a first filter having a pore size of from 30 to 70 µm and a second filter having a pore size of from 0.45 to 4 µm. The kit comprises the filter tool, of which the second filter is a hydrophobic filter, a dye-containing liquid composition, and a colorimetric means. To measure the number of bacteria in samples, a bacteria-containing sample is introduced into the filter tool in such a manner that the sample is first brought into contact with the first filter, whereby the bacteria are entrapped with the second filter, and the number of bacteria in the sample is determined from the degree of coloration of the second filter.

## Description

### BACKGROUND OF THE INVENTION

### 1. Field of the Invention:

The present invention relates to a coloring composition for microorganisms, a filter tool for entrapping bacteria, and a kit for measuring the number of bacteria. Concretely, the invention includes a first aspect relating to a coloring composition for microorganisms, a method for storing the coloring composition for microorganisms, and a method for coloring microorganisms; a second aspect relating to a kit for entrapping bacteria; and a third aspect relating to a kit for measuring the number of bacteria and a method for measuring the number of bacteria.

The first aspect of the invention relates to a coloring composition for microorganisms, a method for storing the coloring composition for microorganisms, and a method for coloring microorganisms, which are applicable to coloring solutions for measurement of the number of bacteria, and to the method and kit for measuring the number of bacteria of the third aspect of the invention. The coloring composition for microorganisms and the method for coloring microorganisms of the first aspect of the invention can be effectively utilized in diagnostics in which the presence of bacteria in urine is problematic, and in other various fields including metal working oils, paints and foodstuffs in which a problem of spoilage will arise, and environmental studies of analyzing hot springs.

The first aspect is directed to microorganisms, which include, for example, fungi, yeasts and bacteria. The second and third aspects of the invention are directed to bacteria of those microorganisms. Specifically, the second aspect relates to a filter tool for entrapping bacteria, through which animal and vegetable cells existing in a sample can be removed while, at the same time, the intended bacteria can be entrapped by it. The third aspect relates to a kit for measuring the number of bacteria, which comprises the filter tool as the element device, and to a method for rapidly measuring the number of bacteria using the kit.

Since bacteria can be entrapped rapidly and in a simplified manner with the filter tool of the second aspect, the filter tool can be utilized in the kit and method for measuring the number of bacteria of the third aspect.

Using the kit and method for measuring the number of bacteria of the third aspect, anyone not having any high-level skill and professional knowledge can measure, rapidly and in a simplified manner, the number of bacteria in a sample through one-stage filtration. Therefore, the kit and method of the third can be effectively utilized in a wide variety of fields such as those to which the first aspect is applicable, for example, in diagnostics in which the presence of bacteria in urine is problematic and in the field of foodstuffs.

### 2. Background Information:

In the field of urine analysis, in general, the number of bacteria in a urine sample is measured in order to detect any inflammation and to estimate the condition of the patient. For example, when the bacteria in urine have grown much, the urine is diagnosed as bacteriuria, which suggests urinary tract infection. Therefore, the number of bacteria in urine is measured for early medical treatment and for medicine administration. In that case, the measurement of the number of bacteria in a urine sample is often effected through plate culture, and the test result is clarified on the day following the collection of the urine sample. Therefore, this measuring method is problematic in that rapid and appropriate, urgent medical treatment for the disease could not be made.

In the field of foodstuffs, the number of bacteria in food samples is measured in order to know as to whether or not the food has spoiled. For fermented milk and fermented lactic-drink, the number of lactic acid bacteria in those is measured in order to control it therein. In such cases, the number of those bacteria is mostly effected through agar plate culture. However, plate culture requires technical knowledge and special devices of thermostats, and takes 24 to 48 hours before the test results.

For rapidly detecting bacteria, methods of coloring the bacteria followed by counting the colored bacteria has been developed. For example, Japanese Patent Publication No. 56-38196 discloses a method of coloring bacteria with a composition comprising a chelating agent and a dye, in a pH range of about 7.0 or higher. Japanese Patent Application Laid-Open No. 1-124767 discloses a method of coloring bacteria with a dye that is soluble in a buffer in a pH range of from 8 to 12 while exhibiting its function in that pH range. In these methods disclosed, used is an acidic solution for the purpose of removing the excess dye from the colored system. However, they do not refer to the variation in the degree of coloration of the colored bacteria.

As one means of overcoming the defects in the prior art techniques that take many hours before the final test results, the present applicant has disclosed in Japanese Patent Application Laid-Open No. 6-209790 a method for measuring the number of microorganisms in samples, which comprises coloring microorganisms with fuchsine and monoethanolamine, then entrapping the colored microorganisms in a hydrophobic filer, thereafter removing the excess fuchsine, and finally measuring the degree of coloration of the colored bacteria to thereby determine the number of the bacteria in the sample. In this method, it is possible to measure, rapidly and in a simplified manner, the number of bacteria in samples. In this, however, the degree of coloration often varies with the lapse of time. Therefore, it has been desired to further improve this method.

Japanese Patent Application Laid-Open No. 8-140698 discloses a method for rapid measurement of the number of bacteria in samples, which comprises coloring the bacteria with a dye-containing cleaning liquid followed by filtering the colored system through a hydrophobic filter to thereby entrap the colored bacteria in the filter with simultaneously removing the excess dye that passes through the filter. However, also in this method, the degree of coloration often varies with the lapse of time, and it has been desired to further improve this method.

On the other hand, it is known that coloring compositions for microorganisms often fade during storage. If such faded compositions are used for measuring the number of microorganisms through the degree of coloration of the colored microorganisms, it is impossible to obtain correct data. Therefore, a coloring composition for microorganisms, of which the sameness in the coloring ability is ensured for a long period of time (for example, 2 months or longer) after its preparation, has been desired.

According to the method disclosed in Japanese Patent Application Laid-Open No. 8-140698, the number of bacteria in samples can be measured rapidly and in a simplified manner by anyone with neither expert skill nor technical knowledge without using any special equipment.

However, this method is problematic in that, if animal and vegetable cells exist in samples, those cells are also colored, resulting in that it is difficult to correctly measure the number of the bacteria only in the samples. For this, Japanese Patent Application Laid-Open No. 8-140698 discloses the use of pre-filters to entrap animal cells. However, the pore size of the pre-filters disclosed is larger only in some degree than that of the filters used for entrapping bacteria therein, and therefore the use of the pre-filters is problematic in that, if the number of animal cells in samples is large, the pre-filters are often clogged resulting in that the filtration therethrough is not easy.

### SUMMARY OF THE INVENTION

The present invention is to solve the above-mentioned problems in the prior art. Specifically, the first aspect of the invention is to provide a coloring composition for microorganisms, with which the presence and even the number of microorganisms in samples can be measured rapidly and in a simplified manner and which is characterized in that the degree of coloration of the microorganisms colored with it varies little with the lapse of time, and to provide a method for coloring microorganisms with the composition.

Another object of the first aspect is to provide a method for storing such a coloring composition for microorganisms, in which the composition being stored can have the same colorability for a long period of time.

The second aspect of the invention is to provide a filter tool for entrapping bacteria, with which bacteria only can be entrapped rapidly and in a simplified manner from samples containing bacteria along with animal cells or vegetable cells, and which can be utilized in the third aspect of the invention that provides a kit and a method for measuring the number of bacteria in samples.

The third aspect of the invention is to provide a method for measuring the number of bacteria in samples, which does not require any special equipment and expert knowledge and in which the number of bacteria in samples can be measured rapidly, correctly and in a simplified manner through simple one-step filtration even when the samples contain animal cells or vegetable cells in addition to bacteria, and to provide a measuring kit for the method.

We, the present inventors have assiduously studied in order to solve the problems in the prior art, and, as a result, have found that, when a coloring composition comprising a dye and a surfactant and having a pH value falling within a specifically defined range is used for coloring microorganisms, or when a coloring composition comprising a dye and a surfactant is used therefor in a pH condition falling within a specifically defined range, the problems can be solved. In the prior art, one has employed a conventional technique of coloring bacteria in a neutral to basic region while considering only the colorability of bacteria. As opposed to that conventional knowledge, we, the present inventors have unexpectedly found that, when such a coloring composition capable of functioning in a weakly acidic range is used, not only the colorability of microorganisms with the composition is improved but also the degree of coloration of the colored microorganisms is kept unchanged for a long period of time. The first aspect of the invention has been completed on the basis of these findings.

Additionally having considered different sizes of bacteria and animal cells or vegetable cells and the difference in size therebetween, we, the present inventors have further found that a combination of different filters each having a different, specifically-defined pore size is effective for separating animal and vegetable cells from bacteria, with which, therefore, bacteria only can be entrapped. On the basis of this finding, the second and third aspects of the invention have been completed.

Specifically, the first aspect of the invention comprises the following:
(1) A coloring composition for microorganisms, which comprises a dye and a surfactant and is controlled to have a pH falling between 4 and 6.
(2) A coloring composition for microorganisms, which comprises a dye, a surfactant and a salt and is controlled to have a pH falling between 4 and 6.
(3) A method for coloring microorganisms, which comprises contacting microorganisms with a composition comprising a dye and a surfactant in a pH condition ranging between 4 and 6.
(4) A method for coloring microorganisms, which comprises contacting microorganisms with a composition comprising a dye, a surfactant and a salt in a pH condition ranging between 4 and 6.
(5) A method for storing a coloring composition for microorganisms comprising a dye, in which the composition is stored while being controlled to have a pH falling between 4 and 6.
(6) A method for storing a coloring composition for microorganisms comprising a dye and a surfactant, in which the composition is stored while being controlled to have a pH falling between 4 and 6.
(7) A method for storing a coloring composition for microorganisms comprising a dye and a salt, in which the composition is stored while being controlled to have a pH falling between 4 and 6.
(8) A method for storing a coloring composition for microorganisms comprising a dye, a surfactant and a salt, in which the composition is stored while being controlled to have a pH falling between 4 and 6.
   The second aspect of the invention comprises the following:
(9) A filter tool for entrapping bacteria, which comprises a first filter having a pore size of from 30 to 70 µm and a second filter having a pore size of from 0.45 to 4 µm.
   The third aspect of the invention comprises the following:
(10) A kit for measuring the number of bacteria in samples, which comprises a filter tool for entrapping and detecting bacteria comprising a first filter having a pore size of from 30 to 70 µm and a second filter made of a hydrophobic material and having a pore size of from 0.45 to 4 µm, a dye-containing liquid composition, and a colorimetric means.
(11) A method for measuring the number of bacteria in samples, which comprises introducing a colored bacteria-containing sample into a filter tool for entrapping and detecting bacteria comprising a first filter having a pore size of from 30 to 70 µm and a second filter made of a hydrophobic material and having a pore size of from 0.45 to 4 µm, in such a manner that the sample being introduced is first brought into contact with the first filter, to thereby make the bacteria entrapped by the filer tool, and the number of the bacteria in the sample is detected from the degree of coloration of the second filter.

### BRIEF DESCRIPTION OF THE DRAWINGS

Fig. 1 is an explanatory view showing one embodiment of the kit for measuring the number of bacteria of the third aspect of the invention.

Fig. 2 is an explanatory view showing the embodiment of the kit of the third aspect of the invention, as combined with a commercially-available syringe for measuring the number of bacteria in a sample.

In those drawings, A is a filter housing (composed of a filter housing part A1 and a filter housing part A2); A1 is a filter housing part (in which is housed a second filter); A2 is a filter housing part (for collecting a sample therethrough); B is a syringe; D is a dye-containing liquid composition; E is a container into which is partitioned the dye-containing liquid composition; F is a color reference; 1 is a second filter; 2 is a filter support; 3 is a first filter; 4 is an opening for sample introduction; 5 is a dropping bottle; and 6 is a piston.

### DETAILED DESCRIPTION OF THE INVENTION

The coloring composition for microorganisms of the first aspect of the invention indispensably comprises a dye and a surfactant and must be controlled to have a pH falling between 4 and 6. The dye to be in the coloring composition may be any and every one capable of coloring microorganisms. The coloring composition is generally used in the form of its aqueous solution. Therefore, the dye is preferably soluble in water. For example, the dye includes fuchsine, safranine-O, methylene blue, methyl green, crystal violet, gentiana violet, and Victoria Blue B. Of those, preferred are fuchsine, safranine-O, methylene blue and methyl green, especially fuchsine and safranine-O, since they can color microorganisms in different degrees, depending on the concentration of the microorganisms existing in samples, and therefore, using any of them, it is easy to detect the presence or absence of microorganisms in samples and even to measure the number of microorganisms therein.

The concentration of the dye to be in the composition is not specifically defined provided that the dye can color microorganisms sufficiently and that the excess dye can be removed with ease. Depending on the dye to be used, its concentration may be suitably determined. For example, for fuchsine and safranine-O, the dye concentration may be generally between 0.00005 and 0.001 % (w/v), preferably between 0.0002 and 0.0005 % (w/v). If the dye concentration is lower than 0.00005 %, the coloration will be often insufficient; but if it is higher than 0.001 %, the removal of the excess dye will often require complicated washing operation.

In the coloring composition and the coloring method of the first aspect, the surfactant is indispensable in order to remove the excess dye adhered to microorganisms through washing. If coloring compositions containing no surfactant are used, it is difficult to remove the excess dye, often interfering with correct measurement of the number of microorganisms.

It is desirable that the surfactant to be used in the first aspect hardly changes the pH of the coloring composition and the pH of the coloring system. The surfactant is preferably a nonionic surfactant. Especially preferred is Tween 20, which is a nonionic surfactant, as not causing the pH change.

The amount of the surfactant to be in the composition is preferably between 0.001 and 1.0 % (w/v), more preferably between 0.1 and 0.5 %. This is because, if the surfactant content is smaller than 0.001 %, the removal of the excess dye will often be difficult; but if it is larger than 1.0 %, even the dye adhered to microorganisms will often be removed.

If desired, a preservative such as ethanol may be added to the coloring composition. If added, the concentration of the preservative such as ethanol may be from 1 to 15 % (v/v).

The coloring composition of the first aspect must be controlled to have a pH falling between 4 and 6. This is because, if it has a pH of 3 or smaller, the degree of coloration of microorganisms is too low; but if it has a pH of 8 or larger, the degree of coloration of microorganisms is too strong. If the pH of the composition is about 7 or so, the stability of the color of the colored microorganisms will be poor even though the degree of coloration is not problematic.

If the pH of the coloring composition is not controlled to fall between 4 and 6, the storage stability of the composition is poor, often resulting in that the ability of the composition to color microorganisms will vary in a few weeks.

The pH-controlling agent to be in the composition may be any and every one that is not easily decomposed by microorganisms. It includes, for example, inorganic acids and inorganic alkalis. Of those, preferred are diluted hydrochloric acid and diluted sodium hydroxide, as being easily available, inexpensive and easily handlable.

A salt is preferably added to the coloring composition of the first aspect, which facilitates the entrapping of the colored microorganisms. In particular, where the colored microorganisms are entrapped through filtration, samples having a high concentration of microorganisms are often difficult to filter. In that case, therefore, a salt must be added to the coloring composition in order to increase the filterability of the samples.

The salt to be added to the coloring composition is preferably soluble in water, since the composition is generally used in the form of its aqueous solution. In view of their solubility in water, preferred are inorganic salts of alkali metals. Of those, especially preferred are sodium chloride and potassium chloride, as being easily available, inexpensive and easily handlable.

The salt content of the coloring composition is preferably between 0.5 and 3.0 % (w/v), more preferably between 0.5 and 2.0 % (w/v), even more preferably between 0.8 and 2.0 % (w/v). Outside the range, the sample will often be difficult to filtrate.

The coloring composition of the first aspect is generally diluted with water, whereby the dye content and the surfactant content of the thus-diluted composition are adjusted to be predetermined ones.

The coloring composition is prepared from a dye, a surfactant, a diluent and optionally a pH-controlling agent and a salt. In general, those components may be simply mixed to prepare the coloring composition, for which the order of mixing the components is not specifically defined. Preferably, however, a dye, a surfactant and optionally a salt are added to a diluent, and if the pH of the resulting mixture falls outside the range of from 4 to 6, a pH-controlling agent is added to the mixture to thereby control the pH value of the mixture.

Where the coloring composition of the first aspect is stored, it may be stored as a thick solution having a pH of from 4 to 6. Having the defined pH range, even the thick solution of the composition can be stored stably.

More preferably, the coloring composition is stored in the absence of a surfactant. The coloring composition containing no surfactant can be stably stored for 7 months or longer.

The samples to which the first aspect of the invention is applicable are not specifically defined. The first aspect is widely applicable to various samples. For example, the samples include urine; water-soluble metal working oils such as cutting oils, rolling oils and thermal treatment oils; liquid foodstuffs such as liquid flavor enhancers (e.g., soy, sauce), liquid food and drink (e.g., fermented milk, fermented lactic-drink), and liquors (e.g., wine, *sake*); wash liquids (e.g., wash water) used for washing powdery or solid foodstuffs (e.g., vegetables, fish, meat); water-soluble paints; and even water, for example, from rivers, lakes, ponds, hot springs, water tanks and drains. If desired, the samples may be suitably diluted prior to being subjected to the coloring test. Of such samples, those comprising bacteria can be the samples for the third aspect of the invention.

The concentration of the microorganisms to be in those samples is preferably between 1 x 10⁴ and 1 x 10⁷ cells/ml or so. If it is smaller than 1 x 10⁴ cells/ml, the microorganisms could not be colored well; but it larger than 1 x 10⁷ cells/ml, the filtration, if employed, of the samples will be often difficult.

Using the coloring composition of the first aspect of the invention, the microorganisms existing in samples such as those mentioned hereinabove can be colored, whereby the existence of the microorganisms therein can be confirmed and the number of the microorganisms can be measured.

Now, the coloring method of the first aspect of the invention is described hereinunder. The coloring method is characterized in that a composition comprising a dye and a surfactant is contacted with microorganisms in samples in a pH condition ranging between 4 and 6. Embodiments of the coloring method may include a method of adding a dye and a surfactant to a sample followed by adjusting the pH of the resulting mixture; a method of adding a composition comprising a dye and a surfactant to a sample followed by adjusting the pH of the resulting mixture; and a method of adding the coloring composition of the first aspect of the invention to a sample.

In the coloring method, preferably employed is a composition comprising a dye and a surfactant and having a pH falling between 4 and 6, that is, the coloring composition of the first aspect of the invention. This is because the coloring composition of the first aspect has good storage stability and does not require any pH adjustment before and even during its use.

The amount of the coloring composition to be applied to samples is suitably from 2 to 5 times a sample. In general, the amount of the sample to be processed with the composition may well be from 50 to 100 µl. Therefore, for example, when the amount of the sample is 100 µl, the dye-containing liquid composition to be applied to the sample is suitably from 200 to 500 µl. It is unnecessary to use the composition in an amount of larger than 5 times the sample.

Regarding the amount of the coloring composition to be used for measuring the number of microorganisms in samples, it is desirable that the amount of the composition to be applied to standard samples, in which the number of microorganisms existing is known, to prepare a color reference or a calibration curve is the same as that of the composition to be applied to the sample in which the number of microorganisms existing is unknown, in order to minimize the measurement errors.

Now referred to hereinunder are preferred embodiments of the coloring composition and the coloring method of the first aspect of the invention. Where the coloring composition and the coloring method of the first aspect are used to measure the number of microorganisms in samples, it is desirable to remove the excess coloring composition used. To remove it, for example, employable is any of centrifugation or filtration. Preferred is filtration, as being simple and sure to remove the excess coloring composition. In view of the filtration rate, especially preferred is suction filtration or pressure filtration.

As the filter to be used for the filtration, preferred is a filter made of a hydrophobic material (hereinafter referred to as a hydrophobic filter). For example, preferably used is the second filter, which is one element device in the second aspect of the invention to be described in detail hereinunder, for the same reasons as those for the second aspect.

The filtration area (coloring area) of the hydrophobic filter is not specifically defined, provided that the coloration of the area can be easily detected. The shape of the coloring area is not also specifically defined, but in view of the easiness in detecting its coloration, the area is preferably circular. More preferably, the diameter of the circular coloring area is from 1 to 3 mm or so. The total size of the hydrophobic filter is not specifically defined, but must be at least not smaller than the size of the coloring area.

The color of the hydrophobic filter may be determined, depending on the dye to be used, and may be any easily-detectable one. In order to easily detect the degree of its coloration, preferred is a white hydrophobic filter.

Where the coloring composition and the coloring method of the first aspect of the invention are used to measure the number of microorganisms in samples, optionally employable is a pre-filter through which any other components that may be colored with the coloring composition can be removed. For example, when a sample containing bacteria and animal cells that are larger than the bacteria, such as leukocytes, is tested while using such a pre-filter, the pre-filter can pass the bacteria therethrough but entrap such large-sized animal cells therewith. As the pre-filter, preferred is the first filter which is another element device in the second aspect of the invention.

Where the coloring composition and the coloring method of the first aspect of the invention is used to measure the number of bacteria in samples through suction filtration, a mixture of the coloring composition and the bacteria-containing sample is subjected to suction filtration using a hydrophobic filter for detecting bacteria, whereby the colored bacteria are entrapped with the filter and, at the same time, the excess dye is removed through the filter, and the number of the bacteria in the sample can be known from the degree of coloration of the filter.

Thus, from the degree of coloration of the bacteria in the sample, from which the excess dye has been removed, the number of the bacteria in the sample can be determined. To determine it, for example, (1) the most simple method is to visually observe the degree of coloration using a color reference, but (2) a colorimetric method of measuring the optical density (OD) of the colored sample is also employable. For the visual observation (1), for example, not only the degree of coloration of the front surface of the hydrophobic filter, on which bacteria have deposited, but also that of the back surface thereof can be measured. For those (1) and (2), color references for the both surfaces of the filter used and a calibration curve indicating the relationship between the absorbance of a sample and the number of bacteria in the sample shall be prepared.

For the measurement of the number of bacteria in samples according to the visual observation (1), concretely, the degree of coloration of the bacteria existing on the hydrophobic filter used, or that is, the color intensity thereof is compared with the data in a color reference which shall be previously prepared using standard samples each containing a known number of bacteria.

The color reference can be prepared by coloring standard samples, in which the number of bacteria existing is known, and then washing them in accordance with the coloring method mentioned hereinabove, and thereafter photographing the colored hydrophobic filters used to give color pictures; or by coloring filter paper and the like to the same degrees as those of the colored hydrophobic filters; or by printing color of the same degrees on paper.

For example, where the number of bacteria in urine is measured, the color reference for the measurement may be prepared in the manner mentioned below. Bacteria exist even in healthy urine in a low concentration (at most about 10,000 cells/ml). However, if bacteria exist in its urine in an amount of 100,000 cells/ml or more, it is suggested that the case will be suffering from urinary tract infection; and if in an amount of 1,000,000 cells/ml or more, it is decided that the case is suffering from an infectious disease (urinary tract infection). Therefore, a color reference capable of indicating those data shall be prepared. In general, a standard color reference capable of indicating five points, 1) 1,000 cells/ml, 2) 10,000 cells/ml, 3) 100,000 cells/ml, 4) 1,000,000 cells/ml, and 5) 10,000,000 cells/ml will be good, which, however, is not whatsoever limitative.

For the measurement of the number of bacteria in samples according to the colorimetry (2) to measure the optical density (OD) of a sample, concretely, the dye that has colored the bacteria existing on the hydrophobic filter used is dissolved out in an organic solvent, and the degree of coloration of the thus-dissolved solution is determined from its absorbance, and is compared with the calibration curve as previously prepared relative to the degrees of optical density (OD) of standard samples and the known numbers of the bacteria existing in the standard samples. The wavelength of the light to be used for measuring the absorbance may be suitably determined, depending on the dye used. The organic solvent to be used herein includes various alcohols, but ethanol is preferred.

The calibration curve can be prepared, for example, in the manner mentioned below. Where fuchsine is used as the dye, the degrees of coloration of standard samples as diluted to predetermined dilutions are determined from their optical density (OD) as measured at 492 nm, using a microplate reader, while, on the other hand, the same standard samples as diluted to the same predetermined dilutions are separately incubated in agar plate culture, and the number of the bacteria existing in each of the diluted standard sample is calculated from the number of the colonies formed through the culture. From the both data thus obtained, prepared is the intended calibration curve indicating the relationship between the number of the bacteria existing in each of the diluted standard samples and the optical density of each of those samples.

Now, the second aspect of the invention is described in detail hereinunder. The filter tool for entrapping bacteria of the second aspect comprises two different filters each having a different specific pore size (first filter and second filter). The pore size of filters as referred to herein is one as obtained through physical measurement according to a bubble point method (JIS K3832) or the like, or through grain retention measurement using a powder having a predetermined grain size according to JIS Z8901 or the like. In the filer tool of the second aspect, the first filter having a larger pore size is so disposed that it is first brought into contact with a sample containing bacteria.

The first filter functions to entrap and remove any other cells and large component such as salts precipitate and trash except bacteria to be measured, such as animal and vegetable cells. The animal and vegetable cells as referred to herein indicate animal cells such as leukocytes and epithelial cells, vegetable cells such as algae, and cells larger than bacteria. The size of animal and vegetable cells varies, depending on the type thereof. In general, the size of leukocytes is from 8 to 15 µm or so, that of epithelial cells is from 20 to 40 µm or so, and that of *Chlorella* cells of green algae, which are vegetable cells, is from 3 to 10 µm or so.

In order to exhibit its function to entrap and remove animal and vegetable cells except bacteria, the first filter must have a pore size falling between 30 and 70 µm. In view of the relationship between the filtration rate through the filter and the ability of the filter to entrap animal and vegetable cells, the pore size of the filter is preferably from 30 to 60 µm. If its pore size is smaller than 30 µm, the filter will be clogged with animal and vegetable cells; but if larger than 70 µm, the filter could not completely entrap leukocytes or the like. In addition, if a filter having such a too large pore size is used as the first filter, the second filter which will be mentioned in detail hereinunder will be clogged, as having a small pore size, to thereby lower the filterability of the filter tool; and if it is used as the element device in the kit of the third aspect of the invention, the number of bacteria as measured with the kit might be incorrect.

The thickness of the first filter is preferably from 1.5 to 7 mm, more preferably from 3 to 6 mm. If its thickness is smaller than 1.5 mm, the filter often could not completely entrap animal and vegetable cells; but if larger than 7 mm, bacteria often could not satisfactorily pass through the first filter, resulting in that the first filter will unfavorably entrap many bacteria. Even if such a thick filter is used as the first filter, the ability of the second filter to entrap bacteria does not lower. However, if such a thick filter is used as the element device in the kit of the third aspect of the invention, the number of bacteria as measured with the kit might be incorrect.

The material of the first filter is not specifically defined, but is preferably selected from polyolefins such as polypropylene, or polyvinyl alcohols, as adsorbing few bacteria. Especially preferred are polyvinyl alcohol filters, as being porous and having high entrappability. More preferred are formal-processed polyvinyl alcohol filters.

The second filter functions to entrap bacteria, and its pore size must be from 0.45 to 4 µm. On the other hand, the size of bacteria is approximately from 1 to 2 µm. The second filter of the invention can entrap bacteria, even though its size is larger than the size of bacteria. However, if its size is larger than 4 µm, the second filter could hardly entrap bacteria; and if smaller than 0.45 µm, the filtration through the filter will be difficult.

The material of the second filter is not specifically defined. The filter may be made of a hydrophilic material, such as cellulose acetate. However, for use in dyeing bacteria and measuring the number of the bacteria, if a filter made of a hydrophilic material is used as the second filter, the filter itself is also colored and the removal of the excess dye is difficult. For these reasons, it is desirable that a hydrophobic filter is used as the second filter for that purpose.

The hydrophobic filter may be made of, for example, polyamides, fluorine polymers such as polytetrafluoroethylene (tetrafluoroethylene resin), polyolefins such as polypropylene, polycarbonates, or glass. Of those, especially preferred are hydrophobic filters made of polytetrafluoroethylene or polypropylene, as facilitating the removal of the excess dye used.

If hydrophilic filters such as nitrocellulose filters are surface-treated to make them hydrophobic, they can be used as hydrophobic filters. For the surface treatment, their surfaces may be coated with any of polyamides, fluorine polymers and polyolefins such as those mentioned above.

The filter tool for entrapping bacteria of the second aspect of the invention comprises the two filters mentioned above, in which the first filter must be so disposed that the bacteria-containing sample is first passed through the first filter and thereafter the bacteria thus having passed therethrough are then entrapped by the second filter. Where the second filter in the filter tool is a hydrophobic filter, the filter tool is favorably used in the kit and the method of the third aspect of the invention.

The filter tool of the second aspect shall indispensably comprise the first filter and the second filter as mentioned above. Preferably, those filters are housed in a housing, which can prevent the environmental region from being soiled with bacteria in the actual use of the filter tool.

The filter housing may be tubular or cylindrical, and may be made of plastics or glass. Its capacity may be suitably determined, for example, depending on the amount of the sample to be applied thereto.

The filter housing may be composed of two connectable, separate elements, in which the second filter is housed in one element while the first filter is in the other element. In this embodiment, the filter housing part to house the first filter therein may be tapered to thereby facilitate the introduction of a sample thereinto, and may have sample graduations put thereon before the position at which the first filter is disposed.

In the same embodiment, the other filter housing part to house the second filter therein may have therein a support for the second filter. The filter support is not specifically defined with respect to its material and shape, provided that it can support the second filter. In general, the filter support may be made of a silicone resin.

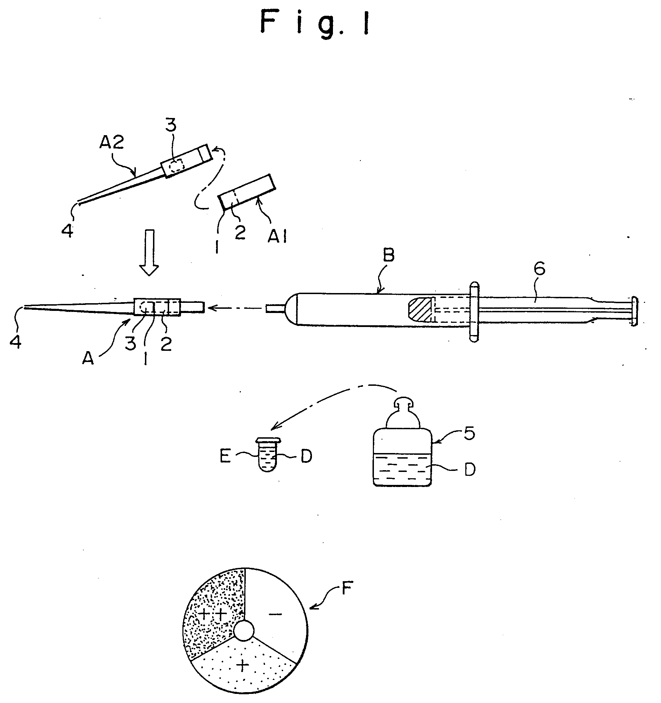

One preferred example of the filter tool of the second aspect of the invention, which is provided with the filter housing of that type, is shown in Fig. 1, in which A indicates the filter housing.

Now described hereinunder is the kit for measuring the number of bacteria of the third aspect of the invention. The kit comprises a filter tool for entrapping and detecting bacteria in samples, a dye-containing liquid composition, and a colorimetric means.

In this kit, the filter tool comprises a first filter having a pore size of from 30 to 70 µm, and a second filter made of a hydrophobic material and having a pore size of from 0.45 to 4 µm. Basically, the structure of the filter tool in this kit is the same as that of the filter tool of the second aspect of the invention mentioned hereinabove.

However, for the kit of the third aspect of the invention, used is a dye-containing liquid composition. Therefore, in this kit, the second filter must be a hydrophobic one for the same reasons as those mentioned above. In addition, in this kit, the second filter functions as a filter for entrapping bacteria and also for detecting bacteria. Concretely, the second filter shall have the function of detecting bacteria while ensuring the interrelationship between the number of the entrapped bacteria and the degree of coloration of the filter itself.

The first filter and the second filter constituting the filter tool in this kit may be the same as those mentioned hereinabove for the second aspect of the invention. Preferably, for example, the first filter may have a thickness of from 1.5 to 7 mm or may be a polyvinyl alcohol filter. The filter housing for those filters may also be the same as that mentioned hereinabove for the second aspect of the invention. However, in the kit of the third aspect of the invention. It is prefer that the first filter and the second filter are separately housed in different filter housing parts for easy detection of the entrapped bacteria.

The dye-containing liquid composition for the kit is to color the entrapped bacteria while acting to wash the filters. Concretely, the composition may comprise a dye capable of coloring bacteria, water or a buffer, and optionally a surfactant.

The, buffer shall be active in a pH range of from 3 to 9, but preferably from 4 to 6 for ensuring the stability of the dye.

As the dye-containing liquid composition, preferably used is the coloring composition of the first aspect of the invention mentioned above.

The colorimetric means to be in the kit of the third aspect is to determine the number of the entrapped bacteria from the degree of coloration of the bacteria-adhered filter. As this means, for example, employable are any means (color references, colorimetry for optical density) such as those referred to hereinabove for the first aspect of the invention.

Now described is the method for measuring the number of bacteria in samples of the third aspect of the invention. The method comprises introducing a colored bacteria-containing sample into a filter tool for entrapping and detecting bacteria comprising a first filter having a pore size of from 30 to 70 µm and a second filter made of a hydrophobic material and having a pore size of from 0.45 to 4 µm, in such a manner that the sample being introduced is first brought into contact with the first filter, to thereby make the bacteria entrapped by the filer tool, and the number of the bacteria in the sample is detected from the degree of coloration of the second filter. As the filter tool in this method, preferably used is the filter tool to be in the kit mentioned above. As the dye-containing liquid composition in this method, preferably used is the coloring composition of the first aspect of the invention mentioned above.

Concretely, in one preferred embodiment of this measuring method, from 70 to 140 µl of a sample is put into a container containing from 200 to 800 µl of the dye-containing liquid composition, or alternatively, the dye-containing liquid composition is put into a container containing the sample, to thereby color the sample. Next, a syringe is fitted into the second filter of the filter tool, and the piston is drawn to thereby introduce the sample into the syringe under suction. If the suction introduction is not easy, a locking member is fitted to the piston by which the back moving of the piston is prevented. After the sample has passed through the second filter, the filter tool is separated from the syringe, then the surface of the second filter is dried, and the degree of coloration of the second filter is compared with a color reference to obtain the number of the bacteria in the sample.

Now, the kit and method for measuring the number of bacteria in samples of the third aspect of the invention are described in detail hereinunder with reference to the drawings attached hereto.

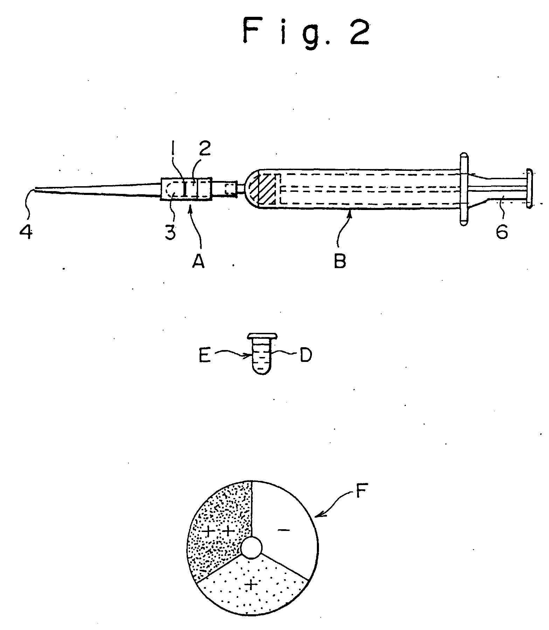

Fig. 1 is an explanatory view showing one embodiment of the kit for measuring the number of bacteria in a sample of the third aspect of the invention. Fig. 2 is an explanatory view showing the embodiment of the kit of the third aspect of the invention, as combined with a commercially-available syringe for measuring the number of bacteria in a sample.

In Fig. 1, the filter housing A has therein a second filter 1, a first filter 3 and a filter support 2. This filter housing A is composed of two elements A1 and A2, in which A1 is a cylindrical filter housing part for the second filter 1 and the filter support 2, and A2 is a tapered filter housing part for the first filter 3.

In the method of the third aspect of the invention, the second filter (hydrophobic filter) 1 is to entrap and detect bacteria. The hydrophobic filter is poorly polar or is not polar, and it is easy to remove the excess dye from it. Therefore, this is preferred as the second filter for entrapping bacteria. The pore size of the second filter 1 must fall between 0.45 and 4 µm as so mentioned hereinabove.

For the filtration area (coloring area) of the second filter 1, the total size thereof and the color thereof, referred to are the same as those for the hydrophobic filter for the first aspect of the invention.

The tip of a syringe B is fittable into one end of the filter housing part A1, and a sample can be introduced into the syringe B under suction via the filter tool. If desired, the kit of the invention may comprise the syringe B. In addition, the it may further comprise a locking member (not shown) for the piston of the syringe.

As in Fig. 1, the tip of the filter housing part A1 (in which the second filter 1 is fitted) is detachably connected with the filter housing part A2 (for collecting a sample therethrough) in which the first filter 3 is fitted. In general, as in Fig. 1 and Fig. 2, the filter housing part A2 is so constructed that the tip of the filter housing part A1 can be partly inserted into one end of the filter housing part A2 in such a manner that the first filter 3 housed in the filter housing part A2 can be kept in contact with the second filter 1 housed in the filter housing part A1.

The filter housing part A2 is tubular or cylindrical with its tip being tapered, and the tip is provided with an opening 4 through which a sample is introduced into the filter housing. If desired, the filter housing part A2 may have sample graduations put thereon. In general, the sample graduations may vary between 50 and 200 µl, while indicating an ordinary amount of a sample.

The first filter 3 to be housed in the filter housing part A2 is to remove animal and vegetable cells. Where a sample containing bacteria and animal cells etc. which are larger than bacteria, such as leukocytes, is applied to the filter tool, the first filter 3 passes the bacteria therethrough but not such large-sized animal cells, and entraps the large cells.

One preferred embodiment of the measuring method of the third aspect of the invention comprises introducing a bacteria-containing sample into the filter housing A under suction. This suction introduction will be described in detail hereinunder.

Precisely, the tip of the filter housing part A1, at which the second filter 1 is housed therein, is fitted into the filter housing part A2 having the first filter 3 as housed therein, in such a manner that the first filter 3 is kept in contact with the second filter 1 in the combined filter housing A.

Next, the syringe B is fitted into the free end of the filter housing part A1 (opposite to the side at which the second filter 1 is housed) to prepare the condition for suction introduction. This constructing process may be effected in any random order. The thus-completed condition is shown in Fig. 2.

As in Fig. 1, the dye-containing liquid composition D is put in a suitable container (for example, the dropping bottle 5) to construct the kit. The kit comprises a suitable container E, into which is partitioned the dye-containing liquid composition D in an amount suitable for one suction filtration, for example, a micro-tube having a volume of approximately from 0.5 to 1.5 ml or so. The dye-containing liquid composition D is partitioned in this container E prior to being introduced into the filter tool for the measurement of the number of bacteria in the sample.

In that manner, a small amount of the dye-containing liquid composition D is previously put into the container E (e.g., micro-tube), and a bacteria-containing sample is added to the container E, using a pipette (not shown), and well mixed therein. As a result, the sample is colored with the composition D.

The ratio of the amount of the dye-containing liquid composition to that of the sample may be the same as that referred to hereinabove for the first aspect of the invention.

In addition, also as has been mentioned hereinabove for the first aspect, it is desirable that the amount of the composition to be applied to standard samples, in which the number of microorganisms existing is known, to prepare a color reference or a calibration curve is the same as that of the composition to be applied to the sample in which the number of microorganisms existing is unknown, in order to minimize the measurement errors.

The mixture of the dye-containing liquid composition and the sample, which has been prepared in the container E, is immediately introduced into the filtration tool, via the opening 4 at the tip of the filter housing part A2 (this is to collect the sample in the filter tool), by drawing the piston 6 in the syringe B, whereby the mixture is filtered under suction through the filter tool.

As a result of this suction filtration, the colored bacteria are entrapped with the second filter, while, at the same time, the excess dye is removed by the second filter.

Precisely, when the piston 6 in the syringe B is drawn for suction, the colored, bacteria-containing sample liquid (this is the mixture of the bacteria-containing sample and the dye-containing liquid composition D) is passed through the second filter, whereupon the bacteria are trapped with the second filter while, at the same time, the excess dye is removed.

Another embodiment for sample collection is also employable herein, which is as follows: For this, the filter housing part A2 is provided with sample graduations put thereon. As in Fig. 2, the filter tool is connected with the syringe B. A predetermined amount of a bacteria-containing sample is introduced into the filter housing part A2, via the opening 4 provided at the tip of the filter housing part A2, by drawing the piston in the syringe B. The amount of the sample to be introduced may be determined by the sample graduations put on the filter housing part A2. Next, the sample thus introduced into the part A2 is ejected out into the container E that contains the dye-containing liquid composition D therein. Thus, the sample is mixed with the composition D, and the bacteria in the sample are colored with the dye. Next, the mixture of the sample and the composition D existing in the container E is introduced into the filter tool by drawing the piston in the syringe B in the same manner as above, whereby the bacteria are entrapped by the second filter.

According to the method of the third aspect of the invention, the colored bacteria are entrapped by the second filter while, at the same time, the excess dye is removed. Thus, the method requires only such simple operation.

In the method of the third aspect, the colored bacteria are entrapped by the second filter 1 while, at the same time, the excess dye is removed, and the number of the bacteria in the sample is determined from the degree of coloration of the second filter 1.

Precisely, after the second filter 1 has entrapped the colored bacteria, the filter housing part A2 is detached from the filter housing part A1, whereby the tip of the filter housing part A1 is exposed outside.

The degree of coloration, or that is, the color intensity of the bacteria deposited on the second filter 1 as housed in the tip of the thus-exposed filter housing part A1 is compared with the color reference F, which was previously prepared from standard samples each containing a known number of bacteria therein, to thereby determine the number of the bacteria in the sample tested. The color reference F in Figs. 1 and 2 is one example of color references applicable to the invention, in which the references, -, + and ++, each indicate the degree of bacteria existing in samples. The color reference F in Figs. 1 and 2 is divided into three portions each indicating the degree of coloration (that is, the number of bacteria), which, however, is not limitative. Employable is any color reference as divided into a larger number of portions.

In the method of the third aspect of the invention, the colored bacteria are entrapped by the second filter while, at the same time, the excess dye is removed, and the number of the bacteria in the sample tested is determined from the degree of coloration of the second filter which is hydrophobic.

To determine the number of bacteria in a sample according to the method of the third aspect of the invention using such a color reference, for example, the degree of coloration, or that is, the color intensity of the bacteria deposited on the second filter is compared with the color reference which is previously prepared from standard samples each containing a known number of bacteria therein.

The color reference for use in the third aspect of the invention can be prepared in the same manner as in the first aspect mentioned hereinabove. Where the number of bacteria in a sample is also determined through colorimetry to measure the optical density (OD) of the sample in accordance with the method of the third aspect, referred to are the same as those in the first aspect.

The method of the third aspect of the invention is especially effective in rapidly determining the total number of bacteria, such as cells of *Escherichia coli*, *Staphylococcus aureus* or *Pseudomonas aeruginosa*, existing in samples.

Now, the invention is described in more detail with reference to the following Examples, which, however, are not intended to restrict the scope of the invention.

First demonstrated hereinunder are embodiments of the first aspect of the invention.

### Reference Example 1:

### Formation of Color References for Measurement of the Number of Bacteria in Urine Using Dye-containing Liquid Composition (Coloring Composition) D1:

Bacteria of *Escherichia coli* (ATCC 11303) and *Staphylococcus aureus* (IFO 3183) were used in the following experiment.

Cells of those strains were inoculated into different bouillon media for each strain, and cultivated at 37°C for 24 hours in static culture. The thus-obtained cultures were used as cell suspensions for standard samples.

On the other hand, bacteria-free urine discharged by a healthy man was collected to prepare a diluent for the cell suspensions.

A varying amount of the diluent was added to the cell suspensions to prepare five standard samples 1) having a cell density of 1,000 cell/ml or so, 2) having a cell density of 10,000 cells/ml or so, 3) having a cell density of 100,000 cells/ml or so, 4) having a cell density of 1,000,000 cells/ml or so, and 5) having a cell density of 10,000,000 cells/ml. The number of the cells in those culture samples was derived from the number of colonies formed through culture of the cells in a CLED agar plate medium at 37°C for 24 hours.

In this experiment, used was the kit of the third aspect of the invention, such as that shown in Fig. 1 and Fig. 2. The sizes of the parts constituting the kit and the dye-containing liquid composition used herein are as follows:
Dye-containing Liquid Composition D1: This comprises 0.0002 % (w/v) of fuchsine, 0.85 % (w/v) of edible salt, and 0.15 % (w/v) of Tween 20 (manufactured by Kanto Chemical Co.), and has a pH of 4.9.
Filter Housing Part A1: This is a polypropylene tube, having an outer diameter of 4.6 mm, an inner diameter of 4 mm and a length of 20 mm.
Filter Housing Part A2 (for sample collection): This is tapered while having sample graduations of 10, 50 and 100 µl put at its tip, and the inner diameter of the mouth at its bottom is 5 mm (manufactured by Quality Co. in US).
Micro-tube (container E for dye-containing liquid composition): This is a 1.5 ml volume, polypropylene tube (manufactured by Quality Co. in US).
Second Filter 1 (hydrophobic filter for detection of bacteria): This is a polytetrafluoroethylene membrane filter having a pore size of 3 µm and a coloring area of 1.5 mm in terms of its diameter.
Filter Support 2: This is a piece of silicone tube, having a length of 7 mm, an outer diameter of 4 mm and an inner diameter of 1.5 mm.
Dropping Bottle 5: This is a 10 ml volume, polypropylene bottle.
First Filter 3 (pre-filter): This is a formal-processed polyvinyl alcohol filter having a diameter of 4 mm, a length of 4 mm and a pore size of 60 µm.

As in Fig. 2, a 3 ml volume syringe B was fitted into one end of the filter housing part A1, while the other end of the filter housing part A1 (at which the hydrophobic filter 1 was fixed) was detachably fitted into the filter housing part A2 having the first filter (pre-filter) 3 therein. 100 µl of the bacteria-containing sample that had been prepared above was introduced into the filter housing part A2, via the opening 4 provided at the tip of the filter housing part A2, by drawing the piston 6 in the syringe B.

Next, the sample thus introduced into the part A2 was ejected out into the container E (micro-tube) containing therein 350 µl (7 or 8 drops) of the dye-containing liquid composition D1.

In this experiment, the sample was collected through the suction of the syringe B. Apart from this mode, a pipette or the like may also be used, in place of the syringe, for collecting the sample. Briefly, two drops (about 100 µl) of the sample may be pipetted into the container E (micro-tube).

Immediately after the addition or pipetting of the sample into the composition D1 in the container E, in which the sample and the composition were mixed, the resulting mixture was introduced into the filter tool through the opening 4 provided at the tip of the part A2, by drawing the piston 6 in the syringe B.

As a result of the suction filtration of the sample mixture through the filter tool, the colored bacteria were entrapped by the hydrophobic filter 1 while, at the same time, the excess dye was removed.

After the colored bacteria were entrapped by the hydrophobic filter 1 in that manner, the filter housing part A2 was separated from the filter housing part A1 as fixed at the tip of the syringe B, whereby the tip of the part A1 was exposed out. Then, the degree of coloration of the colored bacteria existing on the hydrophobic filter 1 as provided at the tip of the filter housing part A1 was visually observed, resulting in the data shown in Table 1 below.

### Reference Example 2:

### Formation of Color References for Measurement of the Number of Bacteria in Urine Using Dye-containing Liquid Composition D2:

Using a dye-containing liquid composition D2, which was the same as the composition D1 in Reference Example 1 except that the fuchsine content was 0.0003 % (w/v), prepared herein were color references for measurement of the number of bacteria in urine, in the same manner as in Reference Example 1. The data obtained are shown in Table 1.

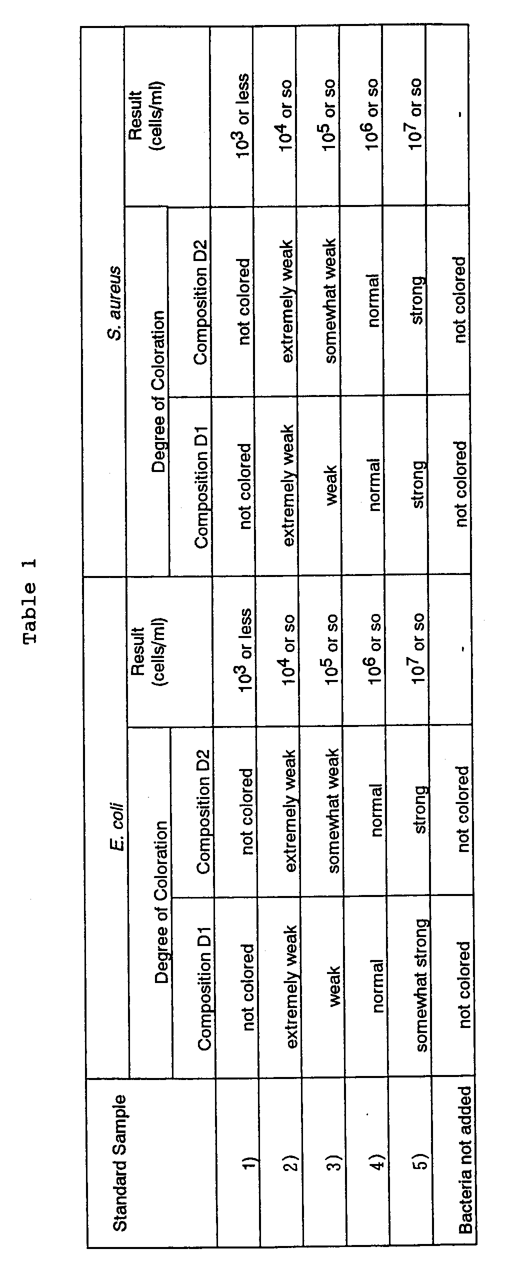

Those colored filters for standard samples were photographed to obtain color pictures, from which were prepared color references. The color references thus prepared in Reference Examples 1 and 2 were sectioned in 5-stage portions, being different from the color reference F shown in Fig. 1 and Fig. 2 which is sectioned in 3-stage portions. In those color references, there was found no significant difference in the degree of coloration between *Escherichia coli* and *Staphylococcus aureus*, and also no significant difference in the degree of coloration between the fuchsine content of 0.0002 % (w/v) and 0.0003 % (w/v).

### Reference Example 3:

### Formation of Color References for Measurement of the Number of Bacteria in Urine Using Dye-containing Liquid Composition D3:

Using a dye-containing liquid composition D3, which was the same as the composition D1 in Reference Example 1 except that 0.0003 % (w/v) of safranine-O was used in place of fuchsine, that the salt content was 1.50 % (w/v) and that the Tween 20 content was 0.17 % (w/v), prepared herein were color references for measurement of the number of bacteria in urine, in the same manner as in Reference Example 1. The data obtained are shown in Table 2.

### Reference Example 4:

Formation of Color References for Measurement of the Number of Bacteria in Urine Using Dye-containing Liquid Composition D4:

Using a dye-containing liquid composition D4, which was the same as the composition D3 in Reference Example 3 except that the safranine-O content was 0.0004 % (w/v), prepared herein were color references for measurement of the number of bacteria in urine, in the same manner as in Reference Example 3. The data obtained are shown in Table 2.

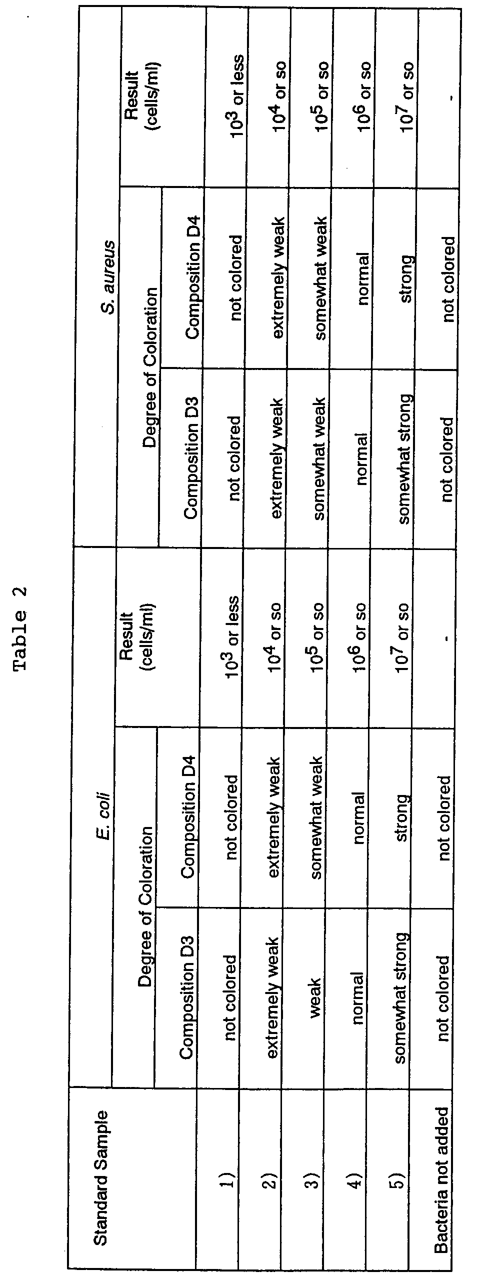

Those colored filters for standard samples were photographed to obtain color pictures, from which were prepared color references. In those color references, there was found no significant difference in the degree of coloration between *Escherichia coli* and *Staphylococcus aureus*, and also no significant difference in the degree of coloration between the safranine-O content of 0.0003 % (w/v) and 0.0004 % (w/v). In addition, in those, there was found no significant difference in the degree of coloration between fuchsine and safranine-O.

### Example 1:

Bacteria of *Staphylococcus aureus* (IFO 3183) were used in this experiment. Precisely, cells of the strain were inoculated into a bouillon medium, and cultivated at 37°C for 24 hours in static culture. The thus-obtained culture was used as a cell suspension for standard samples.

On the other hand, bacteria-free urine discharged by a healthy man was collected to prepare a diluent for the cell suspension.

A predetermined amount of the diluent was added to the cell suspension to prepare a sample having a cell density of 10⁶ cells/ml. The number of the cells in the sample was derived from the number of colonies formed through culture of the cells in a CLED agar plate medium at 37°C for 24 hours.

Fuchsine, edible salt and Tween 20 of 0.0003 % (w/v), 0.85 % (w/v) and 0.15 % (w/v), respectively, were added to a diluted aqueous solution of hydrochloric acid or sodium hydroxide to prepare dye-containing liquid compositions having a controlled pH of from 3.0 to 9.0.

Next, using the same kit as that used in Reference Example 1, the number of bacteria existing in the sample prepared herein (this sample had a cell concentration of 10⁶ cells/ml) was measured in the same manner as in Reference Example 1 except that any of the dye-containing liquid compositions prepared herein to have a controlled pH of from 3.0 to 9.0 was used. Referring to the color references prepared in Reference Example 2, the degree of coloration of each sample was determined, from which was obtained the number of samples in the sample. The data obtained are shown in Table 3 below.

### Example 2:

Dye-containing liquid compositions were prepared in the same manner as in Example 1, except that they comprised 0.0004 % (w/v) of safranine-O in place of fuchsine, 1.50 % (w/v) of edible salt and 0.17 % (w/v) of Tween 20. Using those compositions, the number of bacteria in the urine sample of Example 1 was measured in the same manner as in Example 1. To determine the degree of coloration of each sample tested herein, referred to were the color references prepared in Reference Example 4. From the degree of coloration thus determined, obtained was the number of bacteria in each sample. The data are shown in Table 3.

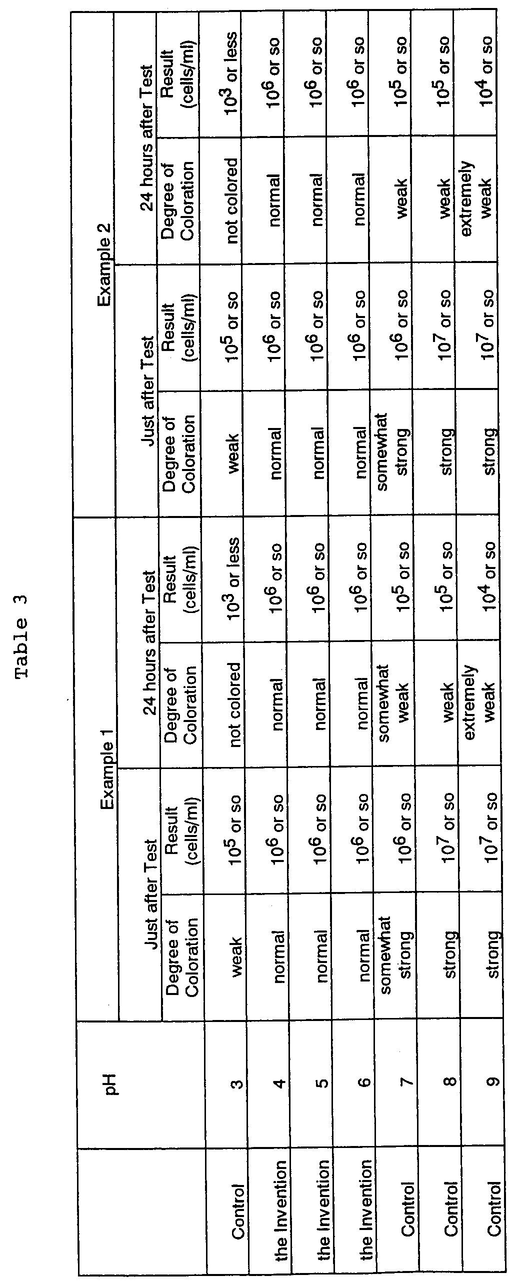

From Table 3, it is known that, when the dye-containing liquid composition having a pH of 3, 8 or 9 was used, the number of bacteria as derived from the number of colonies formed did not correspond to the test data obtained herein, and the degree of coloration of the samples was lowered in 24 hours after the test, indicating the poor reliability of the test data. When the composition having a pH of 7 was used, the test data were nearly correct immediately after the test with a little variation in the degree of coloration of the samples in 24 hours after the test. As opposed to those, when the composition having a pH of from 4 to 6 was used, the test data were good and correct with little variation in the degree of coloration of the samples in 24 hours after the test

### Example 3:

Urine samples were collected from a healthy person and patients suffering from urinary tract infection, in which the number of bacteria was determined in the same manner as in Example 1. Those samples were used in this experiment. On the other hand, fuchsine, and Tween 20 of 0.0003 % (w/v) and 0.15 % (w/v), respectively, were added to a diluted aqueous solution of hydrochloric acid to have a pH of 5.0, to which was added a varying amount of edible salt as in Table 1 to prepare dye-containing liquid compositions. Using any of those compositions, the number of bacteria in each urine sample was measured in the same manner as in Reference Example 1, while referring to the color references prepared in Reference Example 2. In this experiment, in addition, measured was the time for suction filtration of the mixture of the dye-containing liquid composition and the bacteria-containing sample as made by drawing the piston 6 in the syringe. Briefly, to measure the filtration time, the piston was drawn in a settled distance whereupon the time before the complete introduction of the entire amount of the mixture into the syringe was measured in terms of seconds. The sample mixture not completely introduced into the syringe after suction filtration for 10 minutes was determined to be unfilterable. The data obtained are shown in Table 4.

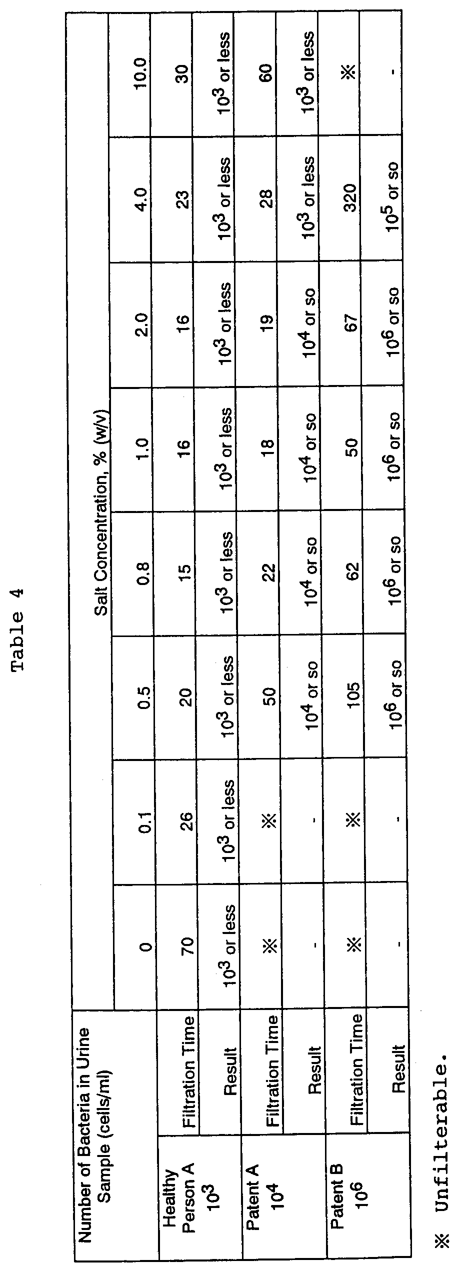

From Table 4, it is known that, when the salt-free, dye-containing liquid composition was used, it was difficult to decide the degree of the infectious uropathy of the patients though their disease itself could be detected. In addition, it was confirmed that, when the salt content of the composition was from 0.5 to 2.0 % (w/v), especially from 0.8 to 2.0 % (w/v), the filtration time for the sample mixture was short and therefore the number of bacteria in the sample was measured correctly.

### Example 4:

In the same manner as in Example 3 except that 0.0004 % (w/v) of safranine-O was used in place of fuchsine, that the Tween 20 content was 0.17 % (w/v) and that the salt content was as in Table 5 below, the filtration time and the number of bacteria in urine samples were determined. To determine the number of bacteria, referred to were the color references prepared in Reference Example 4. The data obtained are shown in Table 5.

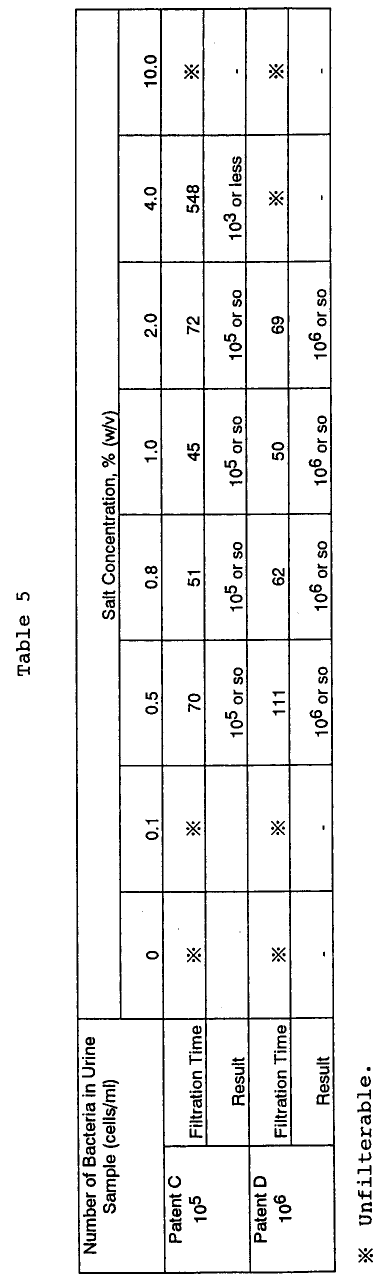

As in Table 5, it was confirmed that the filtration time for the compositions comprising safranine-O and having a salt content of from 0.5 to 2.0 % (w/v), preferably from 0.8 to 2.0 % (w/v) was short and that the compositions were effective in correctly determining the number of bacteria in the urine samples.

### Example 5:

In the same manner as in Example 1, three standard samples each having a different cell density were prepared. Also in the same manner as in Example 1, prepared were dye-containing liquid compositions having a pH of 4.9 and having a varying Tween 20 (surfactant) content as in Table 6 below.

Next, using the same kit as in Reference Example 1, the number of bacteria in those samples was measured in the same manner as above. The degree of coloration of the samples was determined with reference to the color references prepared in Reference Example 2, from which was obtained the number of bacteria in those samples. The data obtained are shown in Table 6.

**Table 6**

| Tween 20 Concentration (%) (w/v) | Number of Bacteria in Standard Sample (cells/ml) | | |
|---|---|---|---|
| | 10³ or less | 10⁵ or so | 10⁷ or so |
| 0 | 10⁶ or so | 10⁷ or so | 10⁷ or so |
| 0.0005 | 10⁵ or so | 10⁶ or so | 10⁷ or so |
| 0.001 | 10⁴ or so | 10⁶ or so | 10⁷ or so |
| 0.01 | 10⁴ or so | 10⁵ or so | 10⁷ or so |
| 0.1 | 10³ or less | 10⁵ or so | 10⁷ or so |
| 0.5 | 10³ or less | 10⁵ or so | 10⁷ or so |
| 1.0 | 10³ or less | 10⁴ or so | 10⁶ or so |
| 5.0 | 10³ or less | 10³ or less | 10⁴ or so |

From Table 6, it is known that, when the composition not containing Tween 20 was used, the excess dye was not removed and correct data could not be obtained except for the samples having a high cell density. However, when the compositions having a too high Tween content were used, the dye was removed too much and correct data could not be obtained except for the samples having a low cell density.

### Example 6:

In the same manner as in Example 1, three standard samples each having a different cell density were prepared. Also in the same manner as in Example 1, prepared was a dye-containing liquid composition having a pH of 4.9. The composition was stored in the dark at room temperature, and its storage stability was determined in the manner mentioned below.

Using the composition in the same kit as in Reference Example 1, the number of bacteria in those samples was measured in the same manner as above. The composition tested was a fresh one (stored for 0 month), one as stored for 2 months, and one as stored for 3 months. The fresh composition was tested 90 times for each sample having the same cell density; while the composition stored for 2 or 3 months was tested 60 times for each sample having the same cell density. The degree of coloration of the samples was determined with reference to the color references prepared in Reference Example 2, from which was obtained the number of bacteria in those samples. The data obtained are shown in Table 7 below.

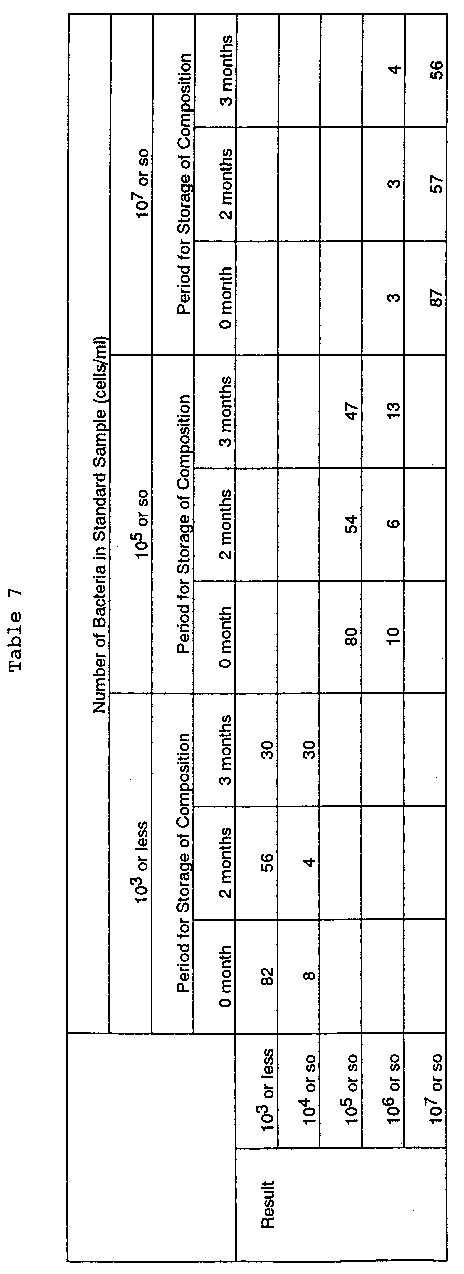

From Table 7, it is known that the dye-containing liquid composition of the invention was still reliable even after having been stored for 2 months to give the same data as those obtained by the use of the fresh sample.

However, when a dye-containing liquid composition having a pH of 7 was prepared in the same manner as in Example 1, and stored in the dark at room temperature for 2 months, it discolored and could not be used in this experiment.

### Example 7:

The same dye-containing liquid composition as in Example 6 except that it did not contain the surfactant was tested for its storage stability in the same manner as in Example 6. In this, however, the surfactant was added to the composition just before its use for the coloration test. The data obtained are shown in Table 8 below.

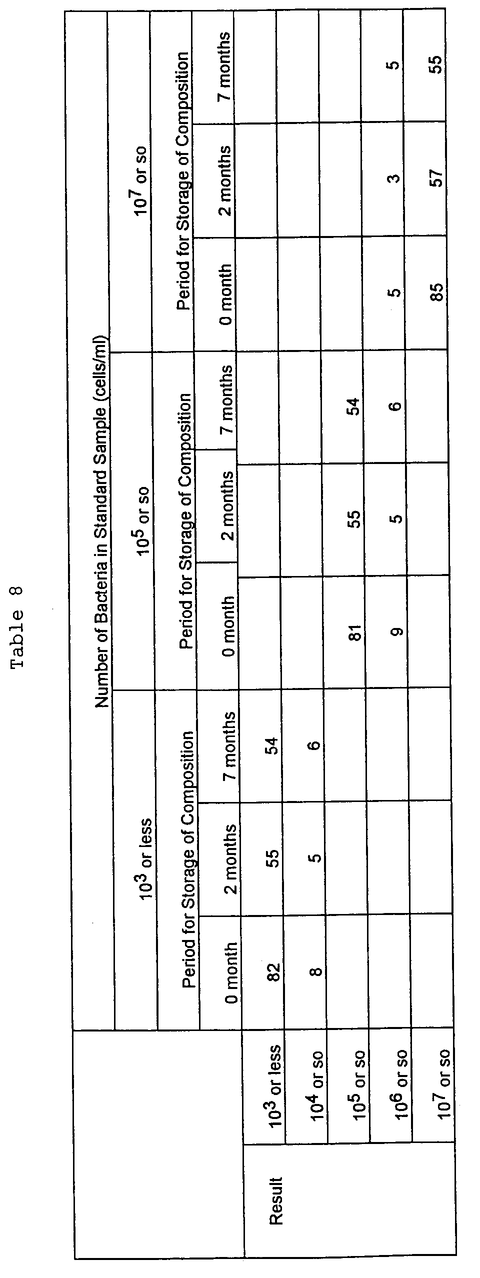

As in Table 8, it was confirmed that the composition as stored in the absence of the surfactant was still reliable even after 7 months to give the same data as those obtained by the use of the fresh sample.

Now hereinunder demonstrated are embodiments of the second and third aspects of the invention.

### Example 8:

Bacteria of *Staphylococcus aureus* (IFO 3183) and *Escherichia coli* (ATCC 11303) were cultivated in different bouillon media at 30°C for 48 hours to prepare cell cultures. These were diluted with sterilized water to prepare cell suspension samples. The number of the cells in each sample was derived from the number of colonies formed through culture of the cells in an agar plate medium (CLED medium). One ml of each sample was filtered through a filter tool comprising a circular, bacteria-entrapping filter (diameter: 13 mm) as housed in a filter housing, whereupon the percentage of the entrapped cells was obtained. As the filter, used herein were a polytetrafluoroethylene (PTFE) filter and a cellulose acetate filter. The number of bacteria existing in the filtrate was obtained from the number of the colonies as formed through the cultivation of the cell-containing filtrate in an agar plate medium (CLED medium). The data obtained are shown in Table 9.

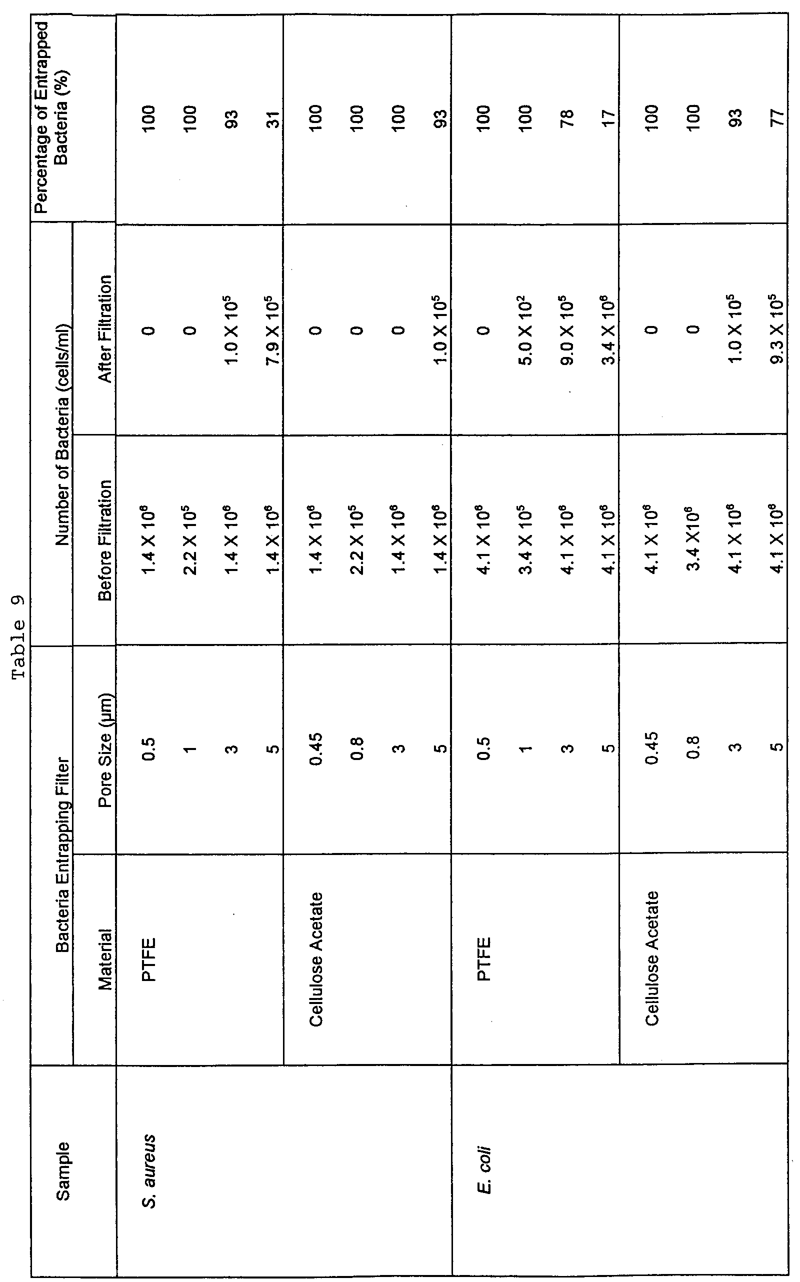

As in Table 9, it was confirmed that the bacteria-entrapping filters having a pore size of from 0.45 to 4 µm entrapped had high bacteria-entrapping ability.

### Example 9:

Cells of the same *E. coli* strain as in Example 8 were cultivated and diluted in the same manner as in Example 8 to prepare bacterial cell suspension samples. The number of cells in each sample and that in each filtrate resulting from the filtration of each sample were obtained also in the same manner as in Example 8.

On the other hand, animal cells of hybridoma SHM-D33 (ATCC 1668) were cultivated in a GIT medium (manufactured by Daigo Nutrient Chemical Co.) for 3 days, and diluted with a PBS buffer (pH 7) to prepare animal cell suspension samples. The number of cells in each sample was counted, using a microscope.

A filter was housed in a tube having an inner diameter of 3 mm, through which one ml of each sample was passed under suction using a syringe to obtain the percentage of entrapped cells. As the filter, used was a formalprocessed polyvinyl alcohol filter having a thickness of 3 mm and having a varying pore size as in Table 10 below. The percentage of entrapped cells in each sample was obtained, while comparing the data before the filtration with those after the filtration that had been obtained in the same manner as that for obtaining the number of animal cells and the number of bacteria in the non-filtered sample.

In this experiment, the filterability of each sample was observed and shown in Table 10, in which "○" indicates that the sample was filtered smoothly, "△" indicates that the complete filtration of the sample needed much time, and " X " indicates that the sample could not be filtered entirely even after a lot of time. The data obtained herein are shown in Table 10.

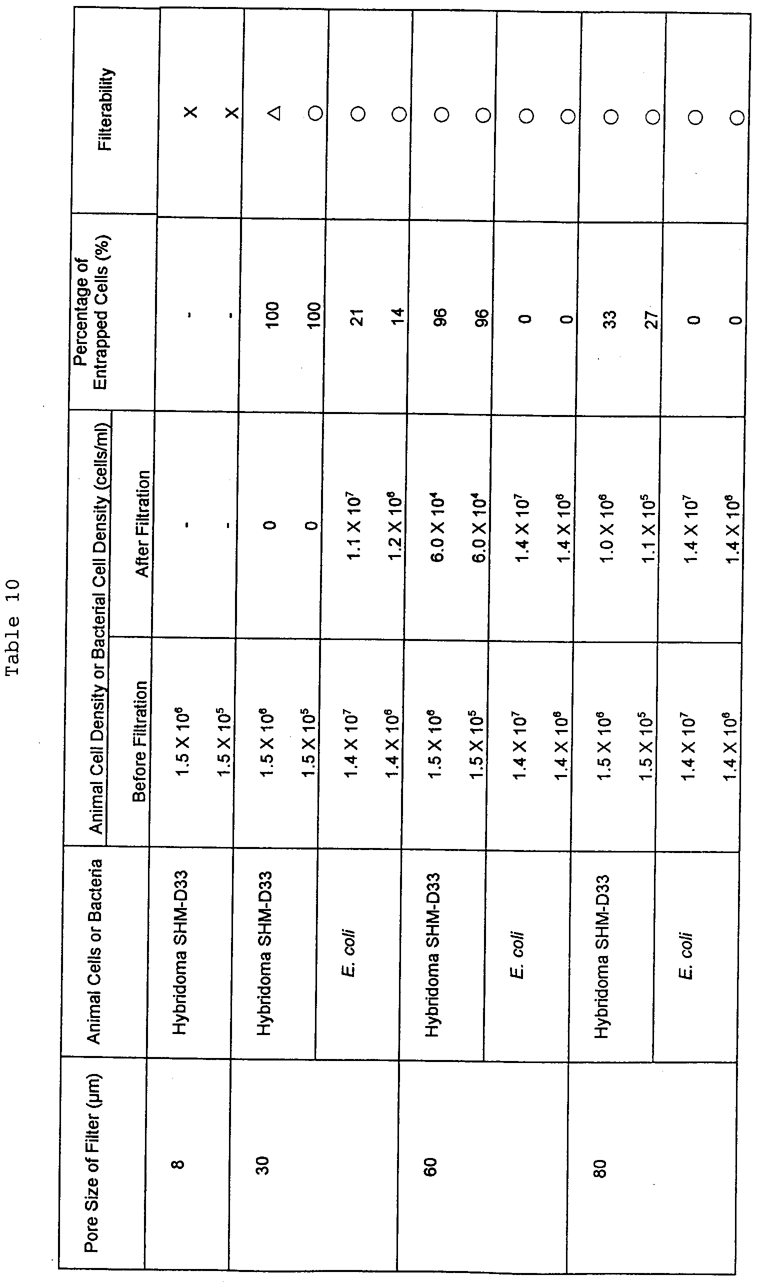

As in Table 10, it was confirmed that the filter having a pore size of from 30 to 70 µm had a high ability to entrap animal cells therewith and a high ability to pass bacterial cells therethrough, while having good filterability.

### Example 10:

The filter having a pore size of 60 µm, which was verified to have good animal cells-entrapping ability, good bacterial cell-passing ability and good filterability in Example 9, was tested for the influence of its thickness on its abilities. Briefly, filters having a varying thickness as in Table 11 below were prepared, and tested in the same manner as in Example 9 using the same samples as in Example 9. The data obtained are shown in Table 11.

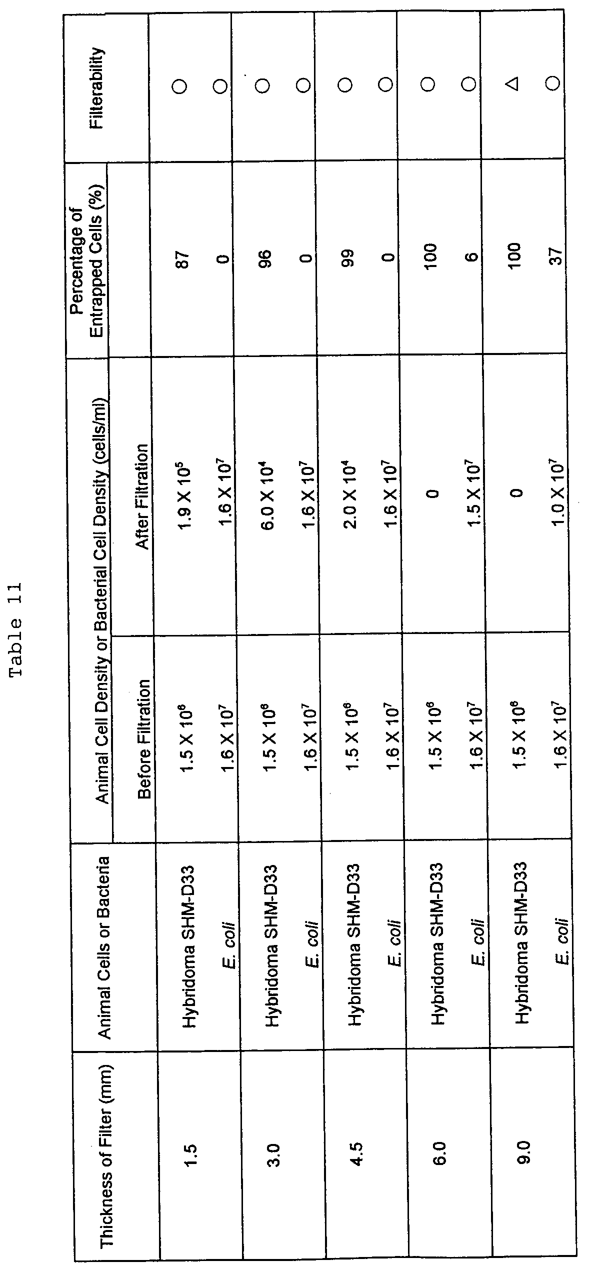

Table 11 suggests that the ability of the filter to entrap hybridoma cells will lower if its thickness is smaller than 1.5 mm while the ability of entrapping cells of *E. coli* will increase if its thickness is larger than 9.0 mm, though the influence of the thickness of the filter on its abilities is smaller than that of the pore size thereof.

### Example 11:

### (1) Formation of Color References for Measurement of the Number of Bacteria in Urine:

Bacteria of *Escherichia coli* (ATCC 11303) and *Staphylococcus aureus* (IFO 3183) were used in the following experiment.

Cells of those strains were inoculated into different bouillon media for each strain, and cultivated at 37°C for 24 hours in static culture. The thus-obtained cultures were used as bacterial cell suspensions for standard samples.

On the other hand, bacteria-free urine discharged by a healthy man was collected to prepare a diluent for the bacterial cell suspensions.

A varying amount of the diluent and a PBS buffer (pH 7) containing cells of hybridoma SHM-D33 (ATCC 1668) were added to the bacterial cell suspensions to prepare five standard samples 1) having a bacterial cell density of 1,000 cell/ml or so, 2) having a bacterial cell density of 10,000 cells/ml or so, 3) having a bacterial cell density of 100,000 cells/ml or so, 4) having a bacterial cell density of 1,000,000 cells/ml or so, and 5) having a bacterial cell density of 10,000,000 cells/ml, in all of which the hybridoma density was 100,000 cells/ml or so. Apart from those, a control sample containing only cells of hybridoma SHM-D33 was prepared. The number of the bacterial cells in those samples was derived from the number of colonies formed through culture of the cells in a CLED agar plate medium at 37°C for 24 hours. The number of the hybridoma cells in those was counted, using a microscope.

In this experiment, used was the kit shown in Fig. 1 and Fig. 2. The sizes of the parts constituting the kit and the dye-containing liquid composition used herein are as follows:
Dye-containing Liquid Composition D5: This comprises 0.0004 % (w/v) of fuchsine, 0.85 % (w/v) of edible salt, and 0.15 % (w/v) of Tween 20 (manufactured by Kanto Chemical Co.), and has a pH of 4.9.
Filter Housing Part A1: This is a polypropylene tube, having an outer diameter of 4.6 mm, an inner diameter of 4 mm and a length of 20 mm.
Filter Housing Part A2: This is a tapered housing, and the inner diameter of the mouth at its bottom is 5 mm (manufactured by Quality Co. in US).
Micro-tube (container E for dye-containing liquid composition): This is a 1.5 ml volume, polypropylene tube (manufactured by Quality Co. in US).
Second Filter 1 (hydrophobic filter for detection of bacteria): This is a polytetrafluoroethylene membrane filter having a pore size of 3 µm and a coloring area of 2.0 mm in terms of its diameter.
Filter Support 2: This is a piece of silicone tube, having a length of 7.5 mm, an outer diameter of 4 mm and an inner diameter of 2.0 mm.
Dropping Bottle 5: This is a 10 ml volume, polypropylene bottle.
First Filter 3: This is a formal-processed polyvinyl alcohol filter having a diameter of 4 mm, a length of 4 mm and a pore size of 60 µm.
Locking Member (not shown): A polypropylene locking member having a length of 55 mm was used.

As in Fig. 2, a 3 ml volume syringe B was fitted into one end of the filter housing part A1, while the other end of the filter housing part A1 (at which the second filter 1 was fixed) was detachably fitted into the filter housing part A2 having the first filter 3 therein.

Using a pipette (not shown), two drops (80 to 100 µl) of the bacteria-containing sample was put into the container E (micro-tube) which had previously contained 350 µl (7 or 8 drops) of the dye-containing liquid composition D5, and mixed.

Immediately after this, the resulting mixture in the container E was introduced into the filter tool through the opening 4 provided at the tip of the part A2, by drawing the piston 6 in the syringe B, whereby the mixture was filtered under suction through the filter tool. During the suction filtration, the back moving of the piston 6 was prevented by the locking member.

As a result of the suction filtration of the mixture through the filter tool, the colored bacteria were entrapped by the second filter 1 while, at the same time, the excess dye was removed.

After the colored bacteria were entrapped by the second filter 1 in that manner, the filter housing part A2 was separated from the filter housing part A1, whereby the tip of the part A1 was exposed out. Then, the degree of coloration of the colored bacteria existing on the second filter 1 as provided at the tip of the filter housing part A1 was visually observed, resulting in the data shown in Table 12 below.

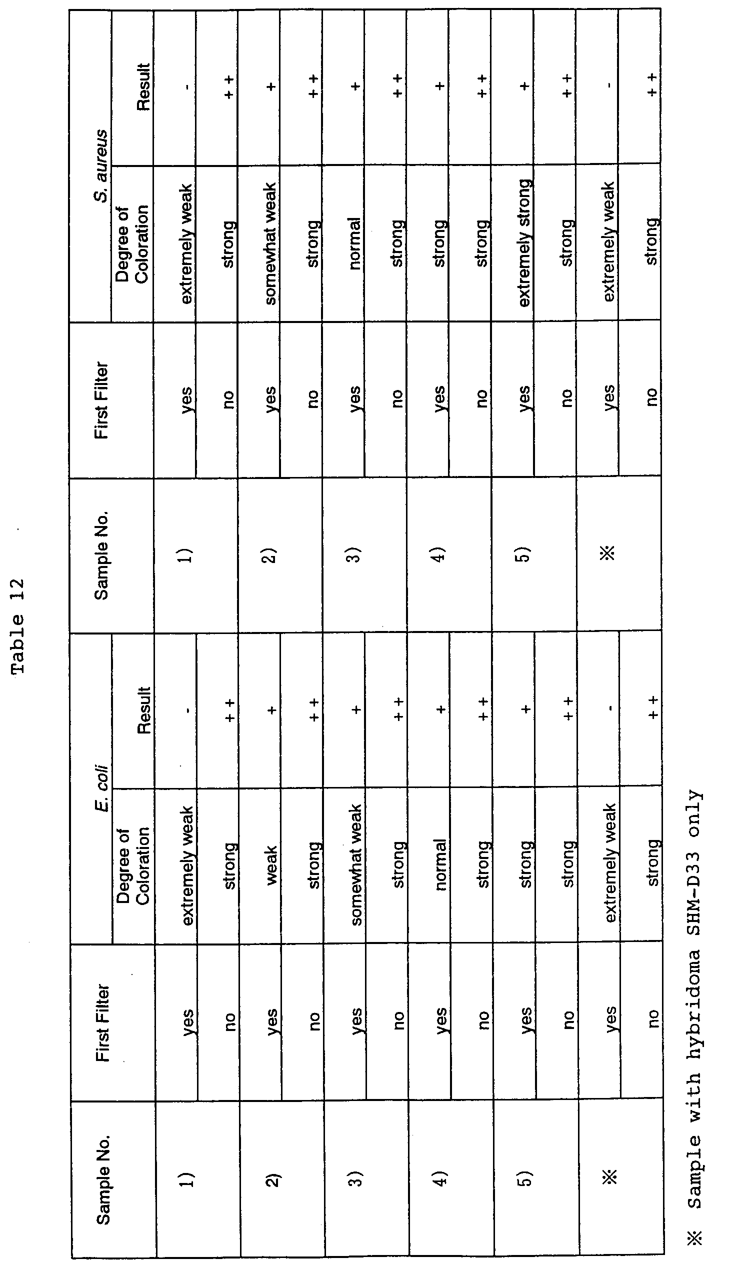

As in Table 12, it is known that the hybridoma cells were entrapped by the second filter in the absence of the first filter, whereby the first filter was colored greatly irrespective of the number of the bacterial cells in the samples. As opposed to this, when the first filter was disposed in the filter tool, the degree of coloration of the second filter increased with the increase in the number of the bacterial cells in the samples. These results support the effect of the invention.

From the degrees of coloration of the second filter for different samples, prepared were 3-staged color references for each strain. In those color references, the stage having a bacterial cell density of smaller than 10⁴ cells/ml was identified as a negative case (-), that having a bacterial cell density of 10⁴ cells/ml or larger was as a positive case (+), and that having a bacterial cell density of 10⁶ cells/ml or larger was as a high positive case (++), in accordance with the Japanese UTI Standard (in which it is ruled that a case having a bacterial cell density of smaller than 10⁴ cells/ml of urine is identified as negative in infectious uropathy and that a case having a bacterial cell density of 10⁴ cells/ml of urine or larger is identified as positive).

### (2) Measurement of the Number of Bacteria in Urine:

Urine samples were collected from healthy persons and patients suffering from urinary tract infection. These were processed in the same manner as in the previous (1), and the color of the second filter for each sample was visually compared with the color references prepared in (1) to determine the number of bacteria in each sample. The data obtained are shown in Table 13 below. For comparison, the numerical data of bacteria in each sample as obtained through culture in a CLED agar plate medium at 37°C for 24 hours are shown in Table 13. In Table 13, "unfilterable" means that the sample could not be filtered at all since the filter tool was clogged.

**Table 13**

| Urine Sample | First Filter | Result from Color Reference | Number of Bacteria Measured (cells/ml) |
|---|---|---|---|
| Healthy Man A | yes | - | 1.8 X 10² |
| | no | + | |
| Healthy Woman B | yes | - | 2.0 X 10³ |
| | no | + | |
| Patient A (man) | yes | + | 7.0 X 10⁴ |
| | no | Unfilterable | |
| Patient B (woman) | yes | + | 1.6 X 10⁵ |
| | no | Unfilterable | |
| Patient C (woman) | yes | + + | 7.9 X 10⁶ |
| | no | unfilterable | |

As in Table 13, when the first filter was not disposed in the filter tool, the sample was unfilterable or gave an erroneous result indicating that a healthy person was erroneously identified as positive. As opposed to this, the filter tool provided with the first filter always gave correct data.

As has been described in detail hereinabove, the coloring composition and the coloring method of the first aspect of the invention are advantageous in that the presence or absence of microorganisms in samples and even the number of microorganisms, if any, in samples can be detected and measured rapidly and in a simplified manner, and that the degree of coloration of colored samples varies little with the lapse of time. Therefore, the composition and the method are favorably used in the kit and method for measuring the number of bacteria in samples of the third aspect of the invention. In addition, the coloring composition of the first aspect has good storage stability.

Using the filter tool for entrapping bacteria of the second aspect of the invention, bacteria only can be entrapped rapidly and in a simplified manner from samples containing bacteria along with animal cells or vegetable cells. Therefore, the filter tool is favorably used in kits and methods for measuring the number of bacteria in samples. Using the kit and method as combined with the filter tool, the number of bacteria in samples can be measured correctly and in a simplified manner. Specifically, the bacteria-entrapping filter tool of the second aspect is especially favorably used in the kit and method for measuring the number of bacteria of the third aspect of the invention.

The kit for measuring the number of bacteria in samples of the third aspect can be handled in an extremely simplified manner, as requiring only one-stage filtration using a syringe. To handle the kit, no skill is needed, and no specific equipment is needed. Therefore, using the kit, the number of bacteria in samples can be measured in any place.

Moreover, since the kit of the third aspect of the invention is extremely simple and inexpensive, while not requiring any specific devices, it is advantageous in that it can be handled by anyone in any place. In particular, since the kit can be handled with ease by anyone with no skill, it can be used for analyzing sea water and other ordinary water, for example, from rivers, lakes, ponds and hot springs, to determine the water quality of those samples. Thus, the kit is effectively used for the protection of the environment. Furthermore, the kit is characterized in that it can be used for bacterial detection in various liquid and solid foods and drinks.

Using the kit, the sample to be analyzed can be collected therein through suction filtration. Therefore, the kit containing the sample to be analyzed therein can be sealed under heat, and can be transported as it is to laboratories with no trouble.

Therefore, the kit and method of the third aspect of the invention can be widely used in various examination fields, for example, for urine analysis in medical diagnosis.

Specifically, in the method of the third aspect, the colored bacteria can be entrapped with the hydrophobic filer while, at the same time, the excess dye can be removed through one-stage suction filtration using a syringe. According to this method, therefore, the number of bacteria in samples can be measured rapidly and in a simplified manner. In general, in this method, the intended data can be obtained within 5 minutes.

In addition, this method does not require any technical skill and knowledge, and it is easy to measure the number of bacteria in samples according to this method.

Moreover, since this method of the third aspect of the invention can clarify the presence or absence of bacteria in samples without cultivating them, it can be utilized for screening specimens for bacterial examinations in hospitals. Therefore, this method is effective for reducing patients' loads and medical expenses.

Furthermore, since the method of the third aspect does not require any specific devices, it can be used even in small-scale hospitals and clinics and even by any ordinary persons with no skill.

Using the method of the third aspect, the number of bacteria in food and drink (such as yogurt) can be measured prior to the distribution thereof in the market. Therefore, this method is effectively utilized for quality control of commercial products.

While the invention has been described in detail and with reference to specific embodiments thereof, it will be apparent to one skilled in the art that various changes and modifications can be made therein without departing from the spirit and scope thereof.

## Claims

1. A coloring composition for microorganisms, which comprises a dye and a surfactant and is controlled to have a pH falling between 4 and 6.

2. The coloring composition for microorganisms as claimed in claim 1, of which the surfactant content is from 0.1 to 0.5 % (w/v).

3. The coloring composition for microorganisms as claimed in claim 2, in which the dye is selected from fuchsine and safranine-O.

4. A coloring composition for microorganisms, which comprises a dye, a surfactant and a salt and is controlled to have a pH falling between 4 and 6.

5. The coloring composition for microorganisms as claimed in claim 4, of which the salt content is from 0.5 to 3.0 % (w/v).

6. The coloring composition for microorganisms as claimed in claim 5, of which the salt content is from 0.8 to 2.0 % (w/v).

7. The coloring composition for microorganisms as claimed in claim 6, in which the salt is selected from sodium chloride and potassium chloride.

8. A method for coloring microorganisms, which comprises contacting microorganisms with a composition comprising a dye and a surfactant in a pH condition ranging between 4 and 6.

9. A method for coloring microorganisms, which comprises contacting microorganisms with a composition comprising a dye and a surfactant and controlled to have a pH falling between 4 and 6.

10. The method for coloring microorganisms as claimed in claim 8 or 9, in which the composition has a surfactant content of from 0.1 to 0.5 % (w/v).

11. The method for coloring microorganisms as claimed in claim 10, in which the dye in the composition is selected from fuchsine and safranine-O.

12. A method for coloring microorganisms, which comprises contacting microorganisms with a composition comprising a dye, a surfactant and a salt in a pH condition ranging between 4 and 6.

13. A method for coloring microorganisms, which comprises contacting microorganisms with a composition comprising a dye, a surfactant and a salt and controlled to have a pH falling between 4 and 6.

14. The method for coloring microorganisms as claimed in claim 12 or 13, in which the composition has a salt content of from 0.5 to 3.0 % (w/v).

15. The method for coloring microorganisms as claimed in claim 14, in which the composition has a salt content of from 0.8 to 2.0 % (w/v).

16. The method for coloring microorganisms as claimed in claim 15, in which the salt in the composition is selected from sodium chloride and potassium chloride.

17. A method for storing a coloring composition for microorganisms comprising a dye, in which the composition is stored while being controlled to have a pH falling between 4 and 6.

18. A method for storing a coloring composition for microorganisms comprising a dye and a surfactant, in which the composition is stored while being controlled to have a pH falling between 4 and 6.

19. A method for storing a coloring composition for microorganisms comprising a dye and a salt, in which the composition is stored while being controlled to have a pH falling between 4 and 6.

20. A method for storing a coloring composition for microorganisms comprising a dye, a surfactant and a salt, in which the composition is stored while being controlled to have a pH falling between 4 and 6.

21. A filter tool for entrapping bacteria, which comprises a first filter having a pore size of from 30 to 70 µm and a second filter having a pore size of from 0.45 to 4 µm.

22. The filter tool for entrapping bacteria as claimed in claim 21, in which the first filter has a thickness of from 1.5 to 7 mm.

23. The filter tool for entrapping bacteria as claimed in claim 22, in which the second filter is a filter made of a material selected from polypropylene and polyfluoroethylene.

24. The filter tool for entrapping bacteria as claimed in claim 23, in which the first filter is a polyvinyl alcohol filter.

25. A kit for measuring the number of bacteria, which comprises a filter tool for entrapping and detecting bacteria comprising a first filter having a pore size of from 30 to 70 µm and a second filter made of a hydrophobic material and having a pore size of from 0.45 to 4 µm, a dye-containing liquid composition, and a colorimetric means.

26. The kit for measuring the number of bacteria as claimed in claim 25, in which the first filter has a thickness of from 1.5 to 7 mm.

27. The kit for measuring the number of bacteria as claimed in claim 25 or 26, in which the colorimetric means is to use color references.

28. The kit for measuring the number of bacteria as claimed in claim 25 or 26, in which the dye-containing liquid composition comprises a dye, a surfactant, and one or more diluents selected from water and buffers.

29. The kit for measuring the number of bacteria as claimed in claim 28, in which the dye is selected from safranine-O, fuchsine, methylene blue and methyl green.

30. A method for measuring the number of bacteria in samples, which comprises introducing a colored bacteria-containing sample into a filter tool for entrapping and detecting bacteria comprising a first filter having a pore size of from 30 to 70 µm and a second filter made of a hydrophobic material and having a pore size of from 0.45 to 4 µm, in such a manner that the sample being introduced is first brought into contact with the first filter, to thereby make the bacteria entrapped by the filer tool, and the number of the bacteria in the sample is detected from the degree of coloration of the second filter.

31. The method for measuring the number of bacteria in samples as claimed in claim 30, in which the first filter has a thickness of from 1.5 to 7 mm.

32. The method for measuring the number of bacteria in samples as claimed in claim 30 or 31, in which the sample is colored with a dye-containing liquid composition.

33. The method for measuring the number of bacteria in samples as claimed in claim 32, in which the dye-containing liquid composition comprises a dye, a surfactant, and one or more diluents selected from water and buffers.

34. The method for measuring the number of bacteria in samples as claimed in claim 33, in which the dye is selected from safranine-O, fuchsine, methylene blue and methyl green.
